# EUROPEAN PATENT SPECIFICATION

(11) **EP 2 591 774 B1**
(45) Date of publication and mention of the grant of the patent: **27.05.2020**
(21) Application number: 11803693.8
(22) Date of filing: 08.07.2011
(51) Int. Cl.: A61K 9/26, A61K 9/00

(54) **ORALLY DISINTEGRATING TABLET**
SCHMELZTABLETTE
COMPRIMÉ SE DÉSINTÉGRANT ORALEMENT

(30) Priority: 09.07.2010 JP 2010156873
(43) Date of publication of application: 15.05.2013
(73) Proprietor: Teijin Pharma Limited, Tokyo 100-0013 (JP)
(72) Inventor: NAKAMURA, Kazuhiro, Iwakuni-shi, Yamaguchi 740-0014 (JP); OGAWA, Teppei, Iwakuni-shi, Yamaguchi 740-0014 (JP)
(74) Representative: Cockerton, Bruce Roger
(86) International application number: PCT/JP2011/065714
(87) International publication number: WO 2012/005359

(56) References cited:
- EP-A1- 1 488 790
- EP-A1- 1 595 533
- WO-A1-2007/055427
- WO-A1-2009/041651
- WO-A2-99/59544
- JP-A- 2001 253 818
- JP-A- 2009 161 495
- JP-A- 2010 502 645
- JP-B1- 3 884 056

## Description

### [TECHNICAL FIELD]

The present invention relates to an intraorally disintegrating tablet comprising an organic acid as a disintegration accelerator.

### [BACKGROUND ART]

Intraorally disintegrating tablets containing organic acids such as ascorbic acids and citric acids are known. Specific examples thereof include, those containing ascorbic acid as an active ingredient (Patent Documents 1-3) and those illustrating the addition of an organic acid as a dissolution agent for an active ingredient in order to ensure absorptivity (Patent Documents 4 and 5). However, none of the above documents describe or suggest contributing to a disintegrating property of intraorally disintegrating tablets. As a technique intended for the disintegrating property thereof, the inclusion of a foaming disintegrant that requires combination with a carbonate is known (Patent Document 6).

On the other hand, the problem of intraorally disintegrating tablets is to simultaneously attain an intraorally disintegrating property, a property of masking unpleasant tastes such as bitterness derived from the active ingredient and an irritating sensation, and a dissolution property intended for the intestinal tract (a dissolution property similar to an ordinary tablet, a controlled dissolution property such as sustained release, etc.), and investigations therefor have been carried out.

As an example thereof, an intraorally disintegrating tablet obtained by compression molding of an active ingredient-containing core particles with an ingredient such as a disintegrant, the particles being coated with a mixture in which a water insoluble polymer such as ethyl cellulose or an enteric polymer such as a methacrylic acid copolymer has been mixed with a disintegrant such as croscarmellose sodium and/or a water permeable ingredient such as hypromellose, is known (Patent Documents 7 and 8) .

While this technique provides a favorable bitterness-masking property and intra-intestinal dissolution property, a disintegrating property immediately after preparation is poor, and thus it was found to have a problem in terms of stable supply of a high quality formulation.

Patent Document 9 discloses an orally disintegrable tablet which comprises (i) fine granules having an average particle diameter of 400 µm or less, which fine granules comprise a composition coated by an enteric coating layer, said composition having 10 weight % or more of an acid-labile physiologically active substance and (ii) an additive. However, this document does not describe an orally disintegrable tablet having a coating layer with a combination of an enteric polymer and a disintegrant.

Patent Document 10 discloses an intraorally rapidly disintegrating tablet, characterized by being produced by tableting cores coated with a pharmaceutical disintegrating agent, wherein the core is a granule containing a water-soluble medicament or containing a medicament and a sugar. However, this document does not disclose a granule obtained by coating a disintegrant-containing particle with a disintegrant.

Patent Document 11 discloses an intraorally rapidly disintegrating tablet, characterized by being produced by tableting cores coated with a pharmaceutical disintegrating agent, wherein the core is a granule containing a water-soluble medicament or containing a medicament and a sugar. However, this document does not disclose a tablet which comprises a water insoluble polymer and/or an enteric polymer in a coating portion of an active ingredient core particle.

Patent Document 12 discloses a solid composition comprising a single crystal of 2-(3-cyano-4-isobutyloxyphenyl)-4-methyl-5-thiazolecarboxylic acid, an excipient, and a disintegrating agent. However, this document does not describe an intraorally disintegrating tablet.

### [RELATED ART DOCUMENTS]

### [PATENT DOCUMENTS]

[Patent Document 1] Japanese Unexamined Patent Publication (Kokai) No. 2009-161495
[Patent Document 2] Japanese Patent No. 3884056
[Patent Document 3] Japanese Unexamined Patent Publication (Kokai) No. 2002-121133
[Patent Document 4] Japanese National Patent Publication (Kohyo) No. 2003-512402
[Patent Document 5] Japanese Unexamined Patent Publication (Kokai) No. 2002-316923
[Patent Document 6] Japanese National Patent Publication (Kokai) No. H5-500956
[Patent Document 7] Japanese National Patent Publication (Kohyo) No. 2003-504324
[Patent Document 8] Japanese Unexamined Patent Publication (Kohyo) No. 2008-214334
[Patent Document 9] WO 99/59544 A2
[Patent Document 10] EP 1 595 533 A1
[Patent Document 11] WO 2007/055427 A1
[Patent Document 12] EP 1 488 790 A1

### [SUMMARY OF INVENTION]

### [PROBLEMS TO BE SOLVED BY THE INVENTION]

An object of the present invention is to provide an intraorally disintegrating tablet that has a property of masking unpleasant tastes such as bitterness derived from the active ingredient and an irritating sensation, and a good dissolution property, and that constantly retains a good intraorally disintegrating property immediately after preparation.

### [MEANS FOR SOLVING THE PROBLEMS]

In view of the above object, the present inventors conducted diligent studies and, as a result, have found that by compression molding of an active ingredient-containing core particle coated with a water insoluble polymer or an enteric polymer together with an organic acid such as ascorbic acid and citric acid to a formulation, a property of masking unpleasant tastes such as bitterness derived from the active ingredient and an irritating sensation, and a good dissolution property can be obtained and good intraorally disintegrating property can be constantly retained immediately after preparation, and thereby have attained the present invention.

That is, the present invention relates to an intraorally disintegrating tablet, in which (i) an active ingredient-containing core particle coated with a coating layer comprising a water insoluble polymer and/or an enteric polymer, and a disintegrant, and (ii) a granule obtained by coating a disintegrant-containing particle with a disintegrant, have been compression molded together with an organic acid.

### [EFFECTS OF THE INVENTION]

In accordance with the present invention, an intraorally disintegrating tablet having a property of masking unpleasant tastes such as bitterness derived from the active ingredient and an irritating sensation, and a good dissolution property and retaining a good intraorally disintegrating property immediately after preparation is provided.

### [DESCRIPTION OF EMBODIMENTS]

The present invention relates to an intraorally disintegrating tablet, in which (i) an active ingredient-containing core particle coated with a coating layer comprising a water insoluble polymer and/or an enteric polymer, and a disintegrant, and (ii) a granule obtained by coating a disintegrant-containing particle with a disintegrant, have been compression molded together with an organic acid.

As used herein an intraorally disintegrating tablet means a tablet capable of being taken by a patient, wherein the tablet disintegrates in the oral cavity, within 60 seconds, preferably within 30 seconds, with only saliva in the oral cavity or with a small amount of water.

The intraorally disintegrating tablet of the present invention may preferably have a practical hardness of 29 N or more, and more preferably of 49 N or more. The preferred dissolution properties of the tablet of the invention are represented by a 60-min dissolution rate of 80% or more, as measured according to the Japanese Pharmacopoeia Paddle method at 50 revolutions per minute, using a pH 6.0 McIlvaine buffer solution.

The core particle in the present invention may contain a fluidizer, such as light anhydrous silicic acid, talc, stearic acid or a metal salt thereof, or the like in addition to the active ingredient.

The particle diameter of the core particle in the present invention is usually 1 to 50 µm, and preferably 3 to 30 µm, as a median diameter as measured by a laser diffraction technique.

The core particle in the present invention is coated with a coating agent containing a water insoluble polymer, an enteric polymer, or a mixture thereof, and a disintegrant. Examples of the water insoluble polymer include ethyl cellulose, aminoalkyl methacrylate copolymer RS and ethyl acrylate/methyl methacrylate copolymer, and examples of the enteric polymer include methacrylic acid/ethyl acrylate copolymer, methacrylic acid/methyl methacrylate copolymer, hydroxypropyl methyl cellulose phthalate, and hydroxypropyl methyl cellulose acetate succinate, etc. Examples of the disintegrant include croscarmellose sodium, low-substituted hydroxypropyl cellulose, and crospovidone. Such a coating agent may contain, in addition to the above, a plasticizer such as macrogol, triethyl citrate, acetylated monoglyceride, triacetin, polysorbate 80, and propylene glycol, an anti-adhesive such as talc, titanium oxide, stearic acid or a metal thereof, sucrose fatty acid ester, and sodium fumarate,and a dissolution rate-controlling substance such as hydroxypropyl cellulose, hydroxypropyl methyl cellulose, carboxymethyl cellulose, polyvinyl alcohol, and polyvinyl pyrrolidone.

The methacrylic acid copolymer for use in the present invention includes, but not particularly limited to, methacrylic acid copolymer LD (for example, trade name: EUDRAGIT L30D55, Evonik), methacrylic acid copolymer L (for example, trade name: EUDRAGIT L100, Evonik), methacrylic acid copolymer S (for example, trade name: EUDRAGIT S100, Evonik).

The content (coating rate) of the coating layer containing these water insoluble polymer and enteric polymer may be usually 2-100 wt%, preferably 5-80 wt%, and more preferably 10-60 wt%, of the core particle.

Examples of the organic acids for use in the present invention include ascorbic acids such as ascorbic acid, sodium ascorbate, potassium ascorbate and ascorbic acid 2-glucoside, citric acid and citrate (collectively referred to as "citric acids", the same hereinafter), fumaric acid and fumarate (fumaric acids), malic acid and malate (malic acids), and tartaric acid and tartrate (tartaric acids), and among them ascorbic acids and citric acids may be preferred.

The content of these organic acids may be usually 0.5-20 wt%, preferably 1-10 wt%, and more preferably 2-5 wt%, of the weight of the tablet.

The tablet of the present invention may comprise additives that are commonly used in tablets as long as they do not specifically affect the bitterness-masking property, the enteric dissolution property, or the disintegrating property. Such additives may include, for example, excipients, binders, lubricants and flavors.

The intraorally disintegrating tablet of the present invention is prepared by compression molding, together with an organic acid, (i) an active ingredient-containing core particle is coated with a layer containing a water insoluble polymer and/or an enteric polymer, and a disintegrant, and (ii) a granule in which a disintegrant-containing particle is coated with a disintegrant .

The active ingredient in the tablet of the present invention include, but not particularly limited to, for example 2-(3-cyano-4-isobutyloxyphenyl)-4-methyl-5-thiazole carboxylic acid (hereinafter referred to as "Compound I").

The intraorally disintegrating tablet of the present invention can be produced without difficulty using a common production equipment or slighly modified production equipment. For example, it can be produced by preparing a granule in which an active ingredient is coated with a coating layer containing a water insoluble polymer and/or an enteric polymer and then by compression molding it together with particles of ascorbic acids or citric acids and one or more pharmaceutically acceptable additives.

### EXAMPLES

### [Example 1]

### (Ascorbic acid is present outside the granule)

After 15.1 g of polysorbate 80 (Nikko Chemicals Co., Ltd.) was added to 522.8 g of purified water, and mixed, 35.3 g of talc (Nikko Pharmaceutical Co., Ltd.) and 12.6 g of croscarmellose sodium (FMC) were added thereto, and then sufficiently stirred (a first solution). Separately from this, a solution prepared by dissolving 1.4 g of sodium hydroxide (Wako Pure Chemical Industries, Ltd.) in 427 g of purified water was added to 380.3 g of methacrylic acid copolymer LD (trade name: EUDRAGIT L30D55, Evonik), and stirred (a second solution). The second solution was added to the first solution and suspended, and sieved to prepare a coating dispersion.

Drug-containing particles were obtained by charging a microparticle coating/granulating apparatus (POWREX CORPORATION, MP-01SFP) with 300 g of Compound I and 15 g of light anhydrous silicic acid (Freund Corporation), and the resulting mixture was sprayed with the above coating dispersion.

Granules were obtained by charging a fluidized bed granulator (POWREX CORPORATION, MP-01) with 870 g of D-mannitol (TOWAKASEI KOGYO, trade name: Mannit P), 40 g of light anhydrous silicic acid (Freund Corporation), and 45 g of crospovidone (ISP, trade name: Polyplasdone XL-10), and the resulting mixture was sprayed with 144 g of purified water. Disintegrant-coated granules were prepared by charging 45 g of crospovidone to powder-coat the above granules.

After 36.2 g of the drug-containing particles, 3.0 g of ascorbic acid (Takeda Pharmaceutical Company Limited), and 2.3 g of calcium stearate (NOF CORPORATION) were added to 108.5 g of the disintegrant-coated granules and mixed together, the resulting mixture was subjected to compression molding with a rotary tableting machine (HATA IRON WORKDS Co., Ltd., HT-AP6SS-U) to form tablets. The molding condition was a tablet weight of 250 mg, and the tableting was performed using a φ8 mm flat punch with a cleavage line to give a hardness of about 78.5 N.

### [Example 2]

### (Citric acid is present outside the granule)

Tablets were formed in the same manner as in Example 1, except that ascorbic acid was replaced with citric acid.

### [Example 3]

### (Sodium ascorbate is present outside the granule)

Tablets were formed in the same manner as in Example 1, except that ascorbic acid was replaced with sodium ascorbate.

### [Example 4]

### (Ascorbic acid 2-glucoside is present outside the granule)

Tablets were formed in the same manner as in Example 1, except that ascorbic acid was replaced with ascorbic acid 2-glucoside.

### [Comparative Example]

### (Ascorbic acid, citric acid, sodium ascorbate, or ascorbic acid 2-glucoside are not present outside the granule)

After 15.1 g of polysorbate 80 (Nikko Chemicals Co., Ltd.) was added to 522.8 g of purified water and mixed, 35.3 g of talc (Nikko Pharmaceutical Co., Ltd.) and 12.6 g of croscarmellose sodium (FMC) were added thereto, and then sufficiently stirred (a first solution). Separately from this, a solution prepared by dissolving 1.4 g of sodium hydroxide (Wako Pure Chemical Industries, Ltd.) in 427 g of purified water was added to 380.3 g of methacrylic acid copolymer LD (trade name: EUDRAGIT L30D55, Evonik), and stirred (a second solution). The second solution was added to the first solution and suspended, and sieved to prepare a coating dispersion.

Compound 1-containing particles were obtained by spraying the above coating dispersion to Compound I and light anhydrous silicic acid in the same manner as in the Examples. Also, disintegrant-coated granules were prepared in the same manner as in the Examples.

After 35.3 g of the drug-containing particles and 2.3 g of calcium stearate (NOF CORPORATION) were added to 112.4 g of the disintegrant-coated granules and mixed together, the mixture was subjected to compression molding in the same molding manner as in the Examples with a rotary tableting machine (HATA IRON WORKDS Co., Ltd., HT-AP6SS-U) to form tablets.

### [Test Example]

Oral disintegration time and masking properties were assessed for the tablets of Examples 1 to 4 and Comparative Example, immediately after preparation. The assessment was performed by two healthy males. That is, the tablets were placed on their tongues and allowed to disintegrate, and the properties of masking the irritation of a base component were rated on the following criteria.
0: Having a clear masking effect and causing no irritation.
1: Having a masking effect and causing little irritation
2: Having a masking effect but causing irritation
3: Having a weak masking effect and causing strong irritation (tolerable)
4: Having no masking effect and causing strong irritation (intolerable)

The results are shown in Table 1.

**[Table 1]**

| | Oral disintegration time (sec) | Masking property |
|---|---|---|
| Example 1 | 18 | 1 |
| Example 2 | 27 | 1 |
| Example 3 | 35 | 1 |
| Example 4 | 26 | 1 |
| Comparative Example | 43 | 2 |

Although Comparative Example is a formulation in which a disintegrant was added, the disintegrating property slowed markedly and the masking property also decreased. It can be seen, however, that in Examples 1 to 4, the addition of an organic acid resulted in improvement of the disintegrating property and the masking property.

### [INDUSTRIAL APPLICABILITY]

The present invention is used in the preparation of intraorally disintegrating tablets.

## Claims

1. An intraorally disintegrating tablet, in which
(i) an active ingredient-containing core particle coated with a layer comprising a water insoluble polymer and/or an enteric polymer, and a disintegrant, and
(ii) a granule obtained by coating a disintegrant-containing particle with a disintegrant,
have been compression molded together with an organic acid.

2. The tablet of claim 1, wherein the content of the organic acid is 1 to 10 wt%.

3. The tablet of claim 1 or 2, wherein the organic acid is ascorbic acids and/or citric acids.

4. The tablet of any one of claims 1 to 3, wherein the water insoluble polymer and/or the enteric polymer is a methacrylic acid copolymer.

5. The tablet of any one of claims 1 to 3, wherein the water insoluble polymer and/or the enteric polymer is ethyl cellulose.

## Patentansprüche

1. Intraoral zerfallende Tablette, in welcher
(i) ein Wirkstoff enthaltendes Kernteilchen beschichtet mit einer Schicht umfassend ein wasserunlösliches Polymer und/oder ein enterisches Polymer, und ein Zerfallsmittel, und
(ii) ein Granulat erhalten durch Beschichten eines Zerfallsmittel enthaltenden Teilchens mit einem Zerfallsmittel,
zusammen mit einer organischen Säure formgepresst wurden.

2. Tablette von Anspruch 1, wobei der Gehalt der organischen Säure 1 bis 10 Gew.-% ist.

3. Tablette von Anspruch 1 oder 2, wobei die organische Säure Ascorbinsäuren und/oder Zitronensäuren ist.

4. Tablette nach einem der Ansprüche 1 bis 3, wobei das wasserunlösliche Polymer und/oder das enterische Polymer ein Methacrylsäure-Copolymer ist.

5. Tablette nach einem der Ansprüche 1 bis 3, wobei das wasserunlösliche Polymer und/oder das enterische Polymer Ethylzellulose ist.

## Revendications

1. Comprimé se désintégrant dans la cavité buccale, dans lequel
(i) une particule de cœur contenant un principe actif, enrobée d'une couche comprenant un polymère insoluble dans l'eau et/ou un polymère entérique, et un délitant, et
(ii) un granule obtenu par enrobage d'une particule contenant un délitant avec un délitant,
ont été moulées par compression conjointement avec un acide organique.

2. Comprimé selon la revendication 1, dans lequel la teneur en l'acide organique est de 1 à 10 % en poids.

3. Comprimé selon la revendication 1 ou 2, dans lequel l'acide organique est l'acide ascorbique et/ou l'acide citrique.

4. Comprimé selon l'une quelconque des revendications 1 à 3, dans lequel le polymère insoluble dans l'eau et/ou le polymère entérique est un copolymère d'acide méthacrylique.

5. Comprimé selon l'une quelconque des revendications 1 à 3, dans lequel le polymère insoluble dans l'eau et/ou le polymère entérique est l'éthylcellulose.
